(19) Europäisches Patentamt
European Patent Office
Office européen des brevets

(11) Veröffentlichungsnummer : **0 061 023**
**B1**

(12) # EUROPÄISCHE PATENTSCHRIFT

(45) Veröffentlichungstag der Patentschrift : **08.02.84**

(51) Int. Cl.³ : **C 12 P 7/62**

(21) Anmeldenummer : **82101560.9**

(22) Anmeldetag : **01.03.82**

(54) **Verfahren zur enzymatischen Herstellung von Estern und Laktonen.**

(30) Priorität : **10.03.81 DE 3108927**

(43) Veröffentlichungstag der Anmeldung : **29.09.82 Patentblatt 82/39**

(45) Bekanntmachung des Hinweises auf die Patenterteilung : **08.02.84 Patentblatt 84/06**

(84) Benannte Vertragsstaaten : **CH DE FR GB IT LI NL**

(56) Entgegenhaltungen :
**FR-A- 1 406 122**
**CHEMICAL ABSTRACTS; Band 91, Nr. 11, 10. September 1979, Seite 609, Nr. 89538f, Columbus, Ohio, USA**
**CHEMICAL ABSTRACTS, Band 87, Nr. 19, 7. November 1977, Seite 440, Nr. 150318v, Columbus, Ohio, USA G.J. MOSKOWITZ et al.: "Hydrolysis of animal fat and vegetable oil with Mucor miehei esterase. Properties of the enzyme"**
**PATENTRS ABSTRACTS OF JAPAN, Band 5, Nr. 89(C-58)(761), 10. Juni 1981**

(73) Patentinhaber : **HAARMANN & REIMER GMBH**
**Postfach 138**
**D-3450 Holzminden (DE)**

(72) Erfinder : **Gatfield, Ian, Dr.**
**Ulmenweg 23**
**D-3470 Höxter (DE)**
Erfinder : **Sand, Theodor, Dr.**
**Vogelsang 3**
**D-3450 Holzminden (DE)**

(74) Vertreter : **Dill, Erwin, Dr. et al**
**c/o BAYER AG Zentralbereich Patente Marken und Lizenzen Bayerwerk**
**D-5090 Leverkusen 1 (DE)**

EP 0 061 023 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur enzymatischen Herstellung von Estern und Laktonen

Die vorliegende Erfindung betrifft ein Verfahren zur enzymatischen Herstellung von Estern und Laktonen.

In der JP-A-54 041 385 wird ein Verfahren zur Herstellung von Terpenalkohol-Estern durch Umsetzen von Terpenalkoholen mit Fettsäuren in Gegenwart von ausgewählten Lipasen beschrieben. Im Gegensatz zu der üblichen Umsetzung von Carbonsäuren mit Alkoholen in Gegenwart einer starken Säure bei erhöhter Temperatur verläuft die enzymatische Veresterung unter schonenden Bedingungen bei Raumtemperatur und in Abwesenheit starker Säuren in hohen Ausbeuten. Nachteilig ist die zur Aktivierung der Lipasen benötigte große Wassermenge, nämlich das zehn- bis zwanzigfache Gewicht der eingesetzten Carbonsäure, die die Reaktionsgeschwindigkeit herabsetzt und bei der Aufarbeitung Schwierigkeiten bereitet.

Es wurde überraschend gefunden, daß man auf den Wasserzusatz verzichten kann, wenn als Enzym die Esterase aus Mucor miehei verwendet wird.

Die Erfindung betrifft daher ein Verfahren zur enzymatischen Herstellung von Estern und Laktonen aus Carbonsäuren oder Carbonsäuregemischen der allgemeinen Formel

$$R—CH_2—CH_2—COOH \qquad (I)$$

worin

R ein Wasserstoffatom oder einen geradkettigen oder verzweigten, gesättigten oder ungesättigten Alkylrest mit 1 bis 21 Kohlenstoffatomen darstellt, der gegebenenfalls durch Hydroxy- oder Alkoxygruppen mit 1 bis 10 Kohlenstoffatomen substituiert sein kann, und primären und sekundären Alkoholen mit 1 bis 15 Kohlenstoffatomen, dadurch gekennzeichnet, daß die Umsetzung in Gegenwart der Esterase aus Mucor miehei in Abwesenheit von Wasser erfolgt.

Die Esterase wird durch Fermentation von Mucor miehei gewonnen (Datenblatt der Firma Rapidase, Seclin/Frankr.). Sie ist eine Lipase mit Esteraseaktivität und zur Hydrolyse von pflanzlichen und tierischen Fetten beispielsweise als Esterase 30 000 der Firma Rapidase im Handel erhältlich.

Als Carbonsäuren der allgemeinen Formel I kommen insbesondere gesättigte Fettsäuren wie Propionsäure, Buttersäure, Capronsäure, Caprylsäure, Caprinsäure, Laurinsäure, Myristinsäure, Palmitinsäure, Stearinsäure und Arachinsäure, und ungesättigte Fettsäuren wie Dec-9-ensäure, Ölsäure, Linolsäure, Linolensäure und Arachidonsäure, und verzweigte Fettsäuren wie Isopalmitinsäure (14-Methyl-pentadecansäure) sowie hydroxy-substituierte Carbonsäuren wie 4-Hydroxy-buttersäure und 15-Hydroxy-pentadecansäure in Frage. Letztere bilden unter den Reaktionsbedingungen Laktone.

Die Carbonsäuren können als reine Verbindungen, aber auch als Gemische wie sie bei der Hydrolyse von natürlichen Fetten wie z. B. Butterfett anfallen, eingesetzt werden.

Als Alkohole kommen beispielsweise gesättigte und ungesättigte, verzweigte und unverzweigte Alkohole mit 1 bis 15 Kohlenstoffatomen wie Methanol, Ethanol, Propan-1-ol, Propan-2-ol, Butan-1-ol, Butan-2-ol, Isobutanol, Hex-3-en-1-ol, Hex-2-en-1-ol, Hexan-1-ol, Oct-7-en-1-ol, Oct-3-en-1-ol, sowie Terpen- und Sesquiterpenalkohole wie Geraniol, Nerol, Citronellol, Farnesol usw. in Frage.

Carbonsäuren und Alkohole werden in einem Molverhältnis von 2 : 1 bis 1 : 6, vorzugsweise in einem Molverhältnis von 1 : 1 bis 1 : 1.1 eingesetzt.

Bei der Veresterung von Propion- und Buttersäure kann es vorteilhaft sein, zur Erhöhung der Reaktionsgeschwindigkeit und der Ausbeute eine höhere Carbonsäure oder den Ester einer höheren Carbonsäure zuzusetzen. Unter höheren Carbonsäuren sind solche mit mehr als 5 Kohlenstoffatomen, insbesondere mit 10 bis 20 Kohlenstoffatomen, zu verstehen. Die Menge an höheren Carbonsäuren sollte 0,5 bis 2 Mol, vorzugsweise 1 Mol, pro Mol Propion- bzw. Buttersäure betragen.

Die erfindungsgemäße Umsetzung kann in der Regel ohne Lösungsmittel durchgeführt werden. Zur Umsetzung von sonst nicht mischbaren Komponenten sowie zur Erhöhung der Rührfähigkeit und bei der Herstellung von Laktonen zur Erzielung eines Verdünnungseffektes kann es vorteilhaft sein, in Gegenwart eines Lösungsmittels zu arbeiten. Als solches kommen Ether wie Diethyläther und Tetrahydrofuran und aromatische Kohlenwasserstoffe wie Benzol und Toluol in Frage.

Die Umsetzung wird vorteilhaft im Temperaturbereich von 0 und 50 °C, vorzugsweise bei Raumtemperatur, ausgeführt.

Die Reaktionszeit hängt von der verwendeten Menge und der Aktivität der Esterase ab und beträgt etwa 24 bis 72 Stunden. Es kann aber, insbesondere bei Reaktionsgemischen, die sich leicht destillativ trennen lassen, vorteilhaft sein, die Reaktion schon früher, z. B. nach 8 Stunden, wenn der Umsatz etwa 40 bis 60 % beträgt, abzubrechen und die wiedergewonnenen Ausgangsmaterialien erneut einzusetzen.

Das Enzym kann in reiner Form oder auf einem Träger, auf dem es chemisch oder physikalisch gebunden ist, eingesetzt werden. Die Menge des Enzyms beträgt, bezogen auf eingesetzte Carbonsäure, 1 bis 30 Gew.-%, vorzugsweise 3 bis 20 Gew.-%, wobei für reine Carbonsäuren 3 Gew.-% ausreichend sind, um eine wirtschaftlich vertretbare Reaktionsgeschwindigkeit zu erzielen, während bei Carbonsäuregemischen etwa 20 Gew.-% einzusetzen sind.

Nach beendeter Umsetzung kann die Esterase durch geeignete Maßnahmen wie Filtrieren oder

Dekantieren abgetrennt und ohne erkennbaren Aktivitätsverlust erneut mehrmals eingesetzt werden.

Während der Umsatz von der Menge und der Aktivität der Esterase sowie der Reaktionszeit abhängt, entspricht die Ausbeute nahezu der Theorie, da die nicht umgesetzten Ausgangsmaterialien unverändert zurückgewonnen und erneut eingesetzt werden können. Der Verlauf der Umsetzung ist überraschend, da bisher davon ausgegangen wurde, daß Lipasen ihre Aktivität nur in wäßrigem Medium entfalten können.

Die erfindungsgemäßen Ester und Lactone können als Riech- und/oder Geschmacksstoffe verwendet werden (Perfume and Flavour Chemicals, S. Arctander (1969)).

## Beispiele

### Beispiel 1

5 g (56,8 mMol) Buttersäure und 35,0 g (227,3 mMol) Geraniol werden mit 750 mg Esterase 30 000 (Fa. Rapidase) versetzt und 72 Stunden bei Raumtemperatur gerührt. Dann wird das Enzym abfiltriert. Aus der Bestimmung der Säurezahl (18,2 mg KOH/g) und der Esterzahl (59,6 mg KOH/g) ergibt sich ein Veresterungsgrad von 76,6 %. Die Destillation des Reaktionsproduktes liefert gaschromatographisch reines Geranyl-n-butyrat. Entsprechende Ergebnisse werden erzielt, wenn anstelle von Geraniol Nerol, Citronellol und Farnesol eingesetzt werden.

### Beispiel 2

7,21 g (50 mMol) Caprylsäure und 15,4 g (100 mMol) Geraniol werden mit 0,43 g Esterase 30 000 analog Beispiel 1 umgesetzt. Die Bestimmung von Säurezahl und Esterzahl ergibt einen Veresterungsgrad von 89,8 %. Die destillative Aufarbeitung liefert gaschromatographisch reines Geranylcaprylat.

### Beispiele 3 bis 5

Analog Beispiel 1 werden 2 g (7 mMol) Ölsäure mit unterschiedlichen Mengen Ethanol und 60 mg Esterase 30 000 versetzt und bei Raumtemperatur gerührt. Zur Bestimmung des Veresterungsgrades werden während der Reaktionszeit Proben entnommen und Säure- und Esterzahl bestimmt. Die Ergebnisse sind in der Tabelle I zusammengefaßt.

Tabelle I

| | | Veresterungsgrad % nach Stunden | | | | | | |
|---|---|---|---|---|---|---|---|---|
| Beispiel | mMol Ethanol | 2,5 | 5 | 8 | 24 | 32 | 48 | 72 |
| 3 | 7 | 16,5 | 34,1 | 51,1 | 79,8 | 84,9 | 84,0 | 90,4 |
| 4 | 14 | 19,3 | 28,6 | 51,7 | 61,3 | 69,2 | 81,4 | 80,0 |
| 5 | 42 | 11,2 | 17,6 | 30,9 | 57,9 | 70,6 | 74,3 | 86,7 |

### Beispiel 6

2,8 g (10 mMol) Ölsäure und 2,96 g (40 mMol) n-Butanol werden mit 60 mg Esterase 30 000 analog Beispiel 1 umgesetzt. Aus Säure- und Esterzahl ergibt sich ein Veresterungsgrad von 83,1 %. Wird anstelle von n-Butanol die gleiche Menge sek.-Butanol eingesetzt, ergibt sich ein Veresterungsgrad von 84,2 %.

### Beispiel 7

5,6 g (20 mMol) Ölsäure und 2,68 g (20 mMol) Oct-3-en-1-ol werden mit 500 mg Esterase 30 000 versetzt und 72 Stunden bei Raumtemperatur gerührt. Die Bestimmung von Säure- und Esterzahl ergibt einen Veresterungsgrad von 90,0 %.

### Beispiel 8

0,88 g (10 mMol) Buttersäure und 1,48 g (20 mMol) n-Butanol werden mit 60 mg Esterase 30 000 analog Beispiel 1 72 Stunden gerührt. Die Bestimmung der Säure- und Esterzahl ergibt einen Veresterungsgrad von 89,4 %.

### Beispiel 9

1,3 g eines Fettsäuregemisches, das durch Hydrolyse von Butterfett erhalten wurde, und 0,46 g Ethanol werden mit 0,26 g Esterase 30 000 versetzt und 72 Stunden bei Raumtemperatur gerührt. Die

Bestimmung des Veresterungsgrades ergibt 98,7 %.

Werden anstelle von Ethanol 0,74 g Isobutanol, 0,88 g Isoamylalkohol oder 0,6 g n-Propanol eingesetzt, so ergeben sich Veresterungsgrade von 87,6 %, 85,6 % und 86,2 %.

Beispiel 10

6,3 g (50 mMol) des Natriumsalzes von 4-Hydroxybuttersäure werden in wenig Wasser gelöst und unter Rühren mit 50 ml 1 N Salzsäure (50 mMol) versetzt, nach 30 Minuten eingefroren und gefriergetrocknet. Das salzhaltige Produkt wird mit 50 ml Toluol versetzt und in Gegenwart von 200 mg Esterase 30 000 72 Stunden bei Raumtemperatur gerührt. Dann wird das Reaktionsgemisch filtriert, das Lösungsmittel abdestilliert und der Rückstand destillativ aufgearbeitet. Man erhält 0,6 g γ-Butyrolacton.

Beispiel 11

4 g Ölsäure und 0,66 g Ethanol werden mit Esterase 30 000 48 Stunden bei Raumtemperatur gerührt. Dann wird das Reaktionsprodukt durch Dekantieren abgetrennt, das Enzym erneut mit Ölsäure und Ethanol versetzt und 48 Stunden bei Raumtemperatur gerührt. Dieser Vorgang wird insgesamt 11 mal wiederholt. Der Veresterungsgrad schwankt dabei zwischen 83,1 und 86,6 %, ohne daß ein Absinken beobachtet werden konnte.

Beispiel 12

Eine Lösung von 1,3 g Esterase in 60 ml Wasser wird mit 15 g eines Kationenaustauschers auf Polystyrolbasis 3 Stunden geschüttelt. Der Überstand wird durch Filtration entfernt und der beladene Ionenaustauscher durch Waschen mit Wasser von überschüssigen Enzymen und dann durch Waschen mit absolutem Ethanol vom Wasser befreit. 6 g des mit Esterase beladenen Ionenaustauschers werden mit 6 g Ölsäure und 1,0 g Ethanol 72 Stunden bei Raumtemperatur gerührt. Dann wird der Katalysator abfiltriert. Die Bestimmung von Säure- und Esterzahl ergibt einen Veresterungsgrad von 81,1 %.

**Ansprüche**

1. Verfahren zur enzymatischen Herstellung von Estern und Laktonen aus Carbonsäuren oder Carbonsäuregemischen der allgemeinen Formel

$$R—CH_2—CH_2—COOH \qquad (I)$$

worin

R ein Wasserstoffatom oder einen geradkettigen oder verzweigten, gesättigten oder ungesättigten Alkylrest mit 1 bis 21 Kohlenstoffatomen darstellt, der gegebenenfalls durch Hydroxy- oder Alkoxygruppen mit 1 bis 10 Kohlenstoffatomen substituiert sein kann, und primären und sekundären Alkoholen mit 1 bis 15 Kohlenstoffatomen, dadurch gekennzeichnet, daß die Umsetzung in Gegenwart der Esterase aus Mucor miehei und in Abwesenheit von Wasser erfolgt.

2. Verfahren nach Anspruch 1, dadurch gekennzeichnet, daß die Umsetzung im Temperaturbereich von 0 bis 50 °C erfolgt.

3. Verfahren nach Anspruch 1 und 2, dadurch gekennzeichnet, daß Carbonsäuren und Alkohole in einem Verhältnis von 2 : 1 bis 1 : 6 eingesetzt werden.

4. Verfahren nach Anspruch 1 bis 3, dadurch gekennzeichnet, daß das Enzym in 1 bis 30 Gew.-%, bezogen auf die eingesetzte Carbonsäure, verwendet wird.

**Claims**

1. Process for the enzymatic preparation of esters and lactones from carboxylic acids or mixtures of carboxylic acids of the general formula

$$R—CH_2—CH_2—COOH \qquad (I)$$

wherein

R represents a hydrogen atom or a straight-chain or branched, saturated or unsaturated alkyl radical which has 1 to 21 carbon atoms and which can be optionally substituted by hydroxyl groups or alkoxy groups with 1 to 10 carbon atoms, and primary and secondary alcohols having 1 to 15 carbon atoms, characterised in that the reaction is effected in the presence of the esterase from Mucor miehei and in the absence of water.

2. Process according to Claim 1, characterised in that the reaction is effected in the temperature

range from 0 to 50 °C.

3. Process according to Claim 1 and 2, characterised in that the carboxylic acids and alcohols are employed in a ratio of 2 : 1 to 1 : 6.

4. Process according to Claim 1 to 3, characterised in that the enzyme is used in a quantity of 1 to 30 % by weight, relative to the carboxylic acid employed.

**Revendications**

1. Procédé pour la fabrication enzymatique d'esters et de lactones à partir d'acides carboxyliques ou de mélanges d'acides carboxyliques de formule

$$R—CH_2—CH_2—COOH \qquad (I)$$

dans laquelle

R représente un atome d'hydrogène ou un reste alkyle saturé ou insaturé à chaîne droite ou ramifiée en $C_1$-$C_{21}$, qui peut éventuellement être substitué par des groupes hydroxy ou des groupes alcoxy en $C_1$-$C_{10}$, et d'alcools primaires et secondaires en $C_1$-$C_{15}$, caractérisé en ce que la réaction s'effectue en présence de l'estérase de Mucor miehei et en l'absence d'eau.

2. Procédé selon la revendication 1, caractérisé en ce que la réaction s'effectue dans l'intervalle de températures de 0 à 50 °C.

3. Procédé selon les revendications 1 et 2, caractérisé en ce que les acides carboxyliques et les alcools sont utilisés dans un rapport molaire de 2 : 1 à 1 : 6.

4. Procédé selon les revendications 1 à 3, caractérisé en ce que l'enzyme est utilisé en quantité de 1 à 30 % en poids par rapport à l'acide carboxylique utilisé.